# EUROPÄISCHE PATENTANMELDUNG

(11) **EP 4 573 962 A1**
(43) Veröffentlichungstag der Anmeldung: **25.06.2025**
(21) Anmeldenummer: 24190700.5
(22) Anmeldetag: 24.07.2024
(51) Int. Cl.: A44C 5/00

(54) **BAND MIT EINER KAMMER FÜR EINEN WIRKSTOFFHALTIGEN BEHÄLTER, INSBESONDERE FÜR CBD PADS, UND DESSEN VERWENDUNG**

(30) Priorität: 22.12.2023 CH 14832023; 22.12.2023 DE 202023107644 U
(71) Anmelder: Green X GmbH, 4147 Aesch (CH)
(72) Erfinder: Chirigione, Francesco, 4147 Aesch (CH); Wiener, Andreas, 4147 Aesch (CH)
(74) Vertreter: Braunpat AG

(57) **Zusammenfassung**

Die vorliegende Erfindung betrifft ein Band (20) umfassend mindestens eine Kammer (10), wobei die mindestens eine Kammer (10) einen ersten Teil (1) aufweist, wobei in die mindestens eine Kammer (10) ein wirkstoffhaltiger Behälter (3) einbringbar und aus der mindestens einen Kammer (10) ausbringbar ist, und wobei der wirkstoffhaltige Behälter (3) in Kontakt bringbar ist mit einem an die mindestens eine Kammer (10) anlegbaren Körperteil eines Menschen oder eines Tiers.

In einer bevorzugten Ausführungsform des erfindungsgemässen Bandes weist die Kammer (10) zusätzlich einen zweiten Teil (2) auf und ist dadurch öffen- und verschliessbar.

Das Band ist insbesondere als Armband für einen Menschen ausgestaltet, und kann insbesondere ein Uhrenarmband oder ein Schmuckstück sein.

Der Behälter (3) ist insbesondere ein Cannabidiol-haltiges Pad.

## Beschreibung

### Technisches Gebiet der Erfindung

Die vorliegende Erfindung betrifft ein Band umfassend mindestens eine Kammer, in die ein wirkstoffhaltiger Behälter ein und ausbringbar ist, und wobei der wirkstoffhaltige Behälter in Kontakt bringbar ist mit einem an die mindestens eine Kammer anlegbaren Körperteil eines Menschen oder eines Tiers. Das Band ist insbesondere als Armband für einen Menschen ausgestaltet, und kann insbesondere ein Uhrenarmband oder ein Schmuckstück sein. Der Behälter ist insbesondere ein Cannabidiol-haltiges Pad, so dass beim Tragen des so ausgestatteten erfindungsgemässen Bandes kontinuierlich Cannabidiol topisch an einen Menschen oder ein Tier verabreicht wird.

### Stand der Technik

Üblicherweise wird ein Band entweder mit einem Wirkstoff beschichtet oder ein Wirkstoff wird in die Polymermatrix eingebaut, aus der das Band besteht. Dies hat zur Folge, dass das Band entsorgt werden muss, sobald der Wirkstoff aus dem Band diffundiert ist.

Aufgabe der vorliegenden Erfindung war es daher, ein Band für die topische Verabreichung eines Wirkstoffes bereit zu stellen, welches nachhaltiger ist als die aus dem Stand der Technik bekannten Bänder.

### Detaillierte Beschreibung der Erfindung

Die Aufgabe wird gelöst durch ein Band gemäss der vorliegenden Erfindung. Die vorliegende Erfindung ist daher auf ein Band gerichtet, welches mindestens eine Kammer mit einem ersten Teil umfasst, wobei in die mindestens eine Kammer ein wirkstoffhaltiger Behälter einbringbar und aus der mindestens einen Kammer ausbringbar ist, und wobei der wirkstoffhaltige Behälter in Kontakt bringbar ist mit einem an die mindestens eine Kammer anlegbaren Körperteil eines Menschen oder eines Tiers. Idealerweise erstreckt sich die mindestens eine Kammer zumindest über einen Teil der Länge des Bandes. Da der wirkstoffhaltige Behälter beliebig oft ausgetauscht werden kann, muss das Band bei erfolgter Diffusion des Wirkstoffes aus dem Behälter nicht entsorgt werden, sondern kann mit einem weiteren, neuen wirkstoffhaltigen Behälter bestückt werden. Dies trägt zur Nachhaltigkeit bei. Ausserdem ist es auf diese Weise auch möglich nacheinander oder abwechselnd unterschiedliche Wirkstoffe topisch zu verabreichen. Weist das erfindungsgemässe Band mehrere Kammern auf, so können gleichzeitig unterschiedliche Wirkstoffe topisch verabreicht werden.

Der erste Teil der mindestens einen Kammer wird im folgenden auch als als Boden oder Kammerboden bezeichnet. In einer bevorzugten Ausführungsform des erfindungsgemässen Bandes ist der wirkstoffhaltige Behälter in diesen ersten Teil der Kammer einklemmbar wie ein Handy in eine Handyhülle, d.h. der erste Teil der Kammer weist idealerweise einen umlaufenden Rand oder entsprechend gestaltete Seitenwände auf, in den/die der wirkstoffhaltige Behälter einklemmbar ist.

In einer ersten bevorzugten Ausführungsform des erfindungsgemässen Bandes ist der wirkstoffhaltige Behälter ein Cannabidiol-haltiges Pad.

In einer zweiten bevorzugten Ausführungsform des erfindungsgemässen Bandes ist die Kammer zweiteilig ausgebildet, und weist zusätzlich zu dem ersten Teil der Kammer noch einen zweiten Teil der Kammer auf, der im folgenden auch als Deckel oder Kammerdeckel bezeichnet wird. Diese zweiteilige Kammer ist öffen- und verschliessbar. Der wirkstoffhaltige Behälter ist hierbei zwischen den ersten Teil und den zweiten Teil der mindestens einen Kammer einklemmbar.

In einer dritten bevorzugten Ausführungsform des erfindungsgemässen Bandes sind der erste Teil der mindestens einen zweiteiligen Kammer und der zweite Teil der mindestens einen zweiteiligen Kammer an mindestens einer Seite lösbar miteinander verbunden. Dadurch kann die mindestens eine Kammer auf einfache Weise geöffnet und geschlossen werden.

In einer vierten bevorzugten Ausführungsform des erfindungsgemässen Bandes ist der Verschluss der mindestens einen zweiteiligen Kammer so ausgestaltet, dass der erste Teil der mindestens einen zweiteiligen Kammer an einer dem zweiten Teil der mindestens einen zweiteiligen Kammer zugewandten Seite erste Rastmittel und der zweite Teil der mindestens einen zweiteiligen Kammer an einer dem ersten Teil der mindestens einen zweiteiligen Kammer zugewandten Seite komplementäre zweite Rastmittel aufweist, wobei die ersten und die zweiten Rastmittel eine Rastverbindung bilden. Diese Rastverbindung zwischen den ersten Rastmitteln und den zweiten Rastmitteln ist bevorzugt formschlüssig. Die Rastverbindung befindet sich an der sogenannten ersten Seite der mindestens einen zweiteiligen Kammer.

Die mindestens eine Kammer kann an jeder Stelle des Bandes angebracht sein. In einer Ausführungsform kann die mindestens eine Kammer über die erste Seite der mindestens einen Kammer, sowie der dieser ersten Seite gegenüber liegenden zweiten Seite der mindestens einen Kammer an dem Band befestigt sein. Die mindestens eine Kammer wird hier also über die zwei kurzen Seitenflächen des Kammerbodens am Band befestigt. Im Prinzip könnte die mindestens eine Kammer so am Band befestigt sein wie beispielsweise ein zeitanzeigendes Element an einem Uhrenarmband befestigt ist.

Ein weiterer Gegenstand der Erfindung ist daher auf ein Set gerichtet, welches mindestens eine erfindungsgemässe Kammer sowie dazu passende Befestigungsmittel enthält, mit denen die mindestens eine Kammer an einem herkömmlichen Uhrenarmband anstelle des zeitanzeigenden Elements befestigt werden kann. Idealerweise enthält das Set unterschiedliche Befestigungsmittel, so dass die mindestens eine Kammer auf unterschiedliche Art und Weise an Uhrenarmbändern befestigbar und mit den Befestigungsmitteln für das zeitanzeigende Element kompatibel ist.

In einer alternativen Ausführungsform kann der erste Teil der mindestens einen zweiteiligen Kammer über die dem zweiten Teil der mindestens einen zweiteiligen Kammer abgewandte Fläche, die sogenannte Grundfläche, am Band befestigt sein. Hierbei können sowohl am Band wie auch an der dem zweiten Teil der mindestens einen zweiteiligen Kammer abgewandten Fläche des ersten Teils der mindestens einen zweiteiligen Kammer jeweils komplementäre erste und zweite Befestigungsmittel angebracht sein. Bei den komplementären ersten und zweiten Befestigungsmitteln kann es sich beispielsweise um einen Klettverschluss handeln. Die mindestens eine Kammer kann alternativ auch am Band angeklebt sein. Ebenso könnte der erste Teil der mindestens einen Kammer so ausgebildet sein, dass ein Durchführen des Bandes am Kammerboden entlang möglich ist.

In einer fünften bevorzugten Ausführungsform des erfindungsgemässen Bandes ist der erste Teil der mindestens einen Kammer so ausgestaltet, dass der wirkstoffhaltige Behälter darin einlegbar ist. In diesem Fall ist der wirkstoffhaltige Behälter als Formkörper ausgebildet, der in den ersten Teil der mindestens einen verschliessbaren Kammer eingepasst wird. So kann man sich hier den ersten Teil der mindestens einen Kammer als Boden mit Seitenwänden vorstellen, auf den der wirkstoffhaltige Behälter gelegt werden kann. Ist die Kammer zweiteilig, so kann man sich den zweiten Teil der mindestens einen Kammer als Deckel vorstellen, so dass beide Teile der Kammer den wirkstoffhaltigen Behälter umschliessen.

In einer sechsten bevorzugten Ausführungsform des erfindungsgemässen Bandes weist der zweite Teil der mindestens einen zweiteiligen Kammer eine Fläche mit mindestens einer Aussparung auf, wobei diese so konfiguiert ist, dass mindestens eine Erhebung auf der dem zweiten Teil der mindestens einen zweiteiligen Kammer zugewandten Fläche des wirkstoffhaltigen Behälters formschlüssig zu ihr ausgebildet ist. Die mindestens eine Erhebung des wirkstoffhaltigen Behälters kann entweder bündig mit der Aussparung sein oder aus dieser herausragen. In jedem Fall ist ein zumindest zeitweiliger Kontakt zwischen einem Körperteil eines Menschen oder eines Tieres gewährleistet, so dass der Wirkstoff von der Haut des Körperteil topisch aufgenommen werden kann.

Statt eines wirkstoffhaltigen Behälters mit entsprechenden Erhebungen könnte auch ein wirkstoffhaltiger Behälter in Form eines Quader, also ohne Erhebungen, in die mindestens eine Kammer gelegt werden. Hierbei sind zwei Alternativen denkbar: Gemäss der ersten Alternative weist der erste Teil der mindestens einen Kammer (= Kammerboden) Erhebungen auf, so dass der Teil des Quaders, der auf den Erhebungen des Bodens zu liegen kommt, bei Verschliessen der mindestens einen Kammer durch die Aussparungen des Kammerdeckels gedrückt wird. Idealerweise sind diese Erhebungen des Kammerbodens formschlüssig zu den Aussparungen des Kammerdeckels ausgebildet. Dies setzt eine gewisse Verformbarkeit des quaderförmigen Wirkstoffbehälters voraus. Gemäss der zweiten Alternative wird ein leicht verformbares, weiches Material für den quaderförmigen wirkstoffhaltigen Behälter verwendet, so dass der wirkstoffhaltige Behälter beim Verschliessen der mindestens einen zweiteiligen Kammer teilweise durch die Aussparungen des Kammerdeckels gequetscht wird und so in Kontakt mit der Haut des Trägers des erfindungsgemässen Bandes kommt.

In einer siebten bevorzugten Ausführungsform des erfindungsgemässen Bandes ist der zweite Teil der mindestens einen zweiteiligen Kammer gegenüber dem ersten Teil der mindestens einen Kammer um einen Winkel von 0 bis mindestens 90° schwenkbar. Dies wird beispielsweise durch ein Gelenkmittel, wie ein Scharnier, ermöglicht, welches den ersten und den zweiten Teil der mindestens einen Kammer miteinander verbindet. Bevorzugt befindet sich das Gelenkmittel an der Seite, welche der ersten Seite gegenüberliegt, an welcher sich die Rastmittel befinden.

Das Band kann ringförmig, sowohl fest als auch flexibel ausgestaltet sein. Darüberhinaus kann es sowohl geschlossen als auch öffen- und verschliessbar sein. Als Verschlüsse können die in der Uhren- und Schmuckbranche üblichen Verschlüsse für Armbänder eingesetzt werden. In einer achten bevorzugten Ausführungsform ist das Band öffen- und verschliessbar, wodurch es ermöglicht wird, das Band eng an ein Körperteil eines Menschen oder eines Tieres anzulegen. Idealerweise ist das Band längenverstellbar, so dass eine individuelle Anpassung an das Körperteils des Trägers/der Trägerin des Bandes möglich ist. Ist das Band ohne Verschluss ausgestaltet, muss es trotzdem so gross sein, dass das Befestigen an einem Körperteil eines Menschen oder eines Tieres möglich ist, welches meist durch Ziehen über das Körperteil, wie z.B. beim Armband das Ziehen über die Hand, erfolgt. Idealerweise hat das Band daher eine gewisse Dehnbarkeit.

In einer weiteren bevorzugten Ausführungsform weist das Band mehrere Bandabschnitte auf, die durch entsprechende Befestigungsmittel miteinander verbunden sind. Hierbei kann die Kammer zwischen zwei Bandabschnitten durch entsprechende Befestigungsmittel an diesen befestigt sein. Insbesondere sind die Befestigungsmittel derart ausgestaltet, dass die einzelnen Bandabschnitte bzw. die Kammer gegeneinander beweglich sind. In diesem Fall können die Befestigungsmittel beispielsweise Scharniere sein.

In einer neunten bevorzugten Ausführungsform dient eine der mindestens einen Kammern als Verschluss des Bandes, wobei das Band nicht geschlossen ist, sondern zwei Enden aufweist, die durch die zweiteilige Kammer lösbar miteinander verbunden werden. Hierbei ist die zweiteilige Kammer an dem einen Ende (auch «erstes Ende» genannt) des Bandes befestigt und das andere Ende (auch «zweites Ende» genannt) des Bandes wird neben dem wirkstoffhaltigen Behälter in die zweiteilige Kammer eingebracht, so dass das zweite Ende des Bandes beim Schliessen der zweiteiligen Kammer, also beim Verrasten des Kammerdeckels mit dem Kammerboden, in der zweiteiligen Kammer eingeklemmt wird, und so das Band geschlossen ist. Bevorzugt ist das zweite Ende des Bandes so ausgestaltet, dass es über den zweiten Teil der zweiteiligen Kammer schiebbar ist. In diesem Fall weist der Kammerdeckel keine Aussparungen auf, sondern der Kammerboden weist Aussparungen auf, durch die hindurch der wirkstoffhaltige Behälter in Kontakt mit der Haut des Trägers des erfindungsgemässen Bandes kommt. D.h. der Kammerdeckel hat in diesem Fall keine Aussparungen, sondern der Kammerboden.

Das erfindungsgemässe Band kann auch mehr als eine Kammer für einen wirkstoffhaltigen Behälter aufweisen. So könnte gemäss einer Ausführungsform das Band zwei Kammern aufweisen, wobei eine Kammer als zweiteilige Kammer wie oben beschrieben als Verschluss des Bandes ausgestaltet ist.

Für das Band sind unterschiedliche Materialen geeignet wie beispielsweise Kautschuk, Naturkautschuk, Silikon, Leder, Metall sowie sämtliche Materialien, die die Fachperson bei der Herstellung von Schmuckarmbändern und Uhrenarmbändern einsetzt.

In einer zehnten bevorzugten Ausführungsform ist das Band ein Armband für einen Menschen.

In einer elften bevorzugten Ausführungsform ist das Armband für einen Menschen als Schmuckstück und/oder Uhrenarmband ausgestaltet. Der Formen- und Farbvielfalt sind hierbei keine Grenzen gesetzt. Insbesondere wird das Uhrenarmband betreffend seiner Breite so ausgestaltet sein, dass es an den gängigen Uhrformaten anbringbar ist. Da das Band meist über seine Länge zumindest teilweise leicht bogenförmig ausgebildet ist, ist auch die mindestens eine Kammer leicht bogenförmig ausgebildet.

In einer zwölften bevorzugten Ausführungsform ist das Band zur Befestigung an einem Körperteil eines nichthumanen Säugetiers ausgestaltet. Das Band ist hierbei insbesondere ein Halsband, ein Beinband oder ein Halfter für ein nichthumanes Säugetier. Das heisst, die Grösse des Bandes ist derart ausgewählt, dass das Band als Halsband oder als Beinband oder als Halfter für ein nichthumanes Säugetier verwendet werden kann. Das nichthumane Säugetier umfasst hierbei insbesondere Haustiere und Nutztiere. Bevorzugt umfasst das nichthumane Säugetier Hunde, Katzen, Rinder, Schafe, Ziegen, Schweine, Kamele und Pferde.

In einer dreizehnten bevorzugten Ausführungsform des erfindungsgemässen Bandes ist in die mindestens eine Kammer ein wirkstoffhaltiger Behälter eingebracht. Schutzgegenstand der vorliegenden Erfindung ist also auch ein Band, in dessen mindestens eine Kammer ein wirkstoffhaltiger Behälter eingebracht ist.

In einer vierzehnten bevorzugten Ausführungsform ist der Behälter gefüllt mit einem pharmazeutischen Wirkstoff und/oder dessen Formulierung. Der pharmazeutische Wirkstoff ist hierbei insbesondere ausgewählt aus Pharmazeutika, welche in der Schmerztherapie und/oder in der Psychotherapie eingesetzt werden, bevorzugt aus Cannabinoiden wie insbesondere Cannabidiol (CBD) und Tetrahydrocannabinol (THC) sowie deren Mischungen und Derivaten wie Ethern und Estern, insbesondere mit C₁₋₁₀-Alkanolen bzw. C₁₋₁₀-Alkansäuren. Besonders bevorzugt ist der pharmazeutische Wirkstoff Cannabidiol. Statt eines pharmazeutischen Wirkstoffs kann auch jede beliebige biologisch aktive Substanz und/oder deren Formulierung in den Wirkstoffbehälter gefüllt oder eingebracht werden.

Jede der oben genannten bevorzugten Ausführungsformen kann mit jeder beliebigen anderen bevorzugten Ausführungsform kombiniert werden, so dass jedwede Kombination auch von der vorliegenden Erfindung umfasst ist.

Ein weiterer Gegenstand der vorliegenden Erfindung ist die Verwendung eines erfindungsgemässen Bandes mit den oben beschriebenen bevorzugten Ausführungsformen zur topischen Verabreichung eines Wirkstoffes an einen Menschen oder ein Tier, wobei die Verwendung insbesondere nicht-medizinisch ist. Durch die mindestens eine speziell ausgestaltete Kammer ist ein Hautkontakt zwischen dem wirkstoffhaltigen Behälter und dem Körperteil eines Menschen oder eines Tieres zumindest zeitweilig gegeben. Durch die Körperwärme wird der Wirkstoff aus dem wirkstoffhaltigen Behälter freigesetzt. Da die mindestens eine Kammer, wenn sie an einem Körperteil eines Menschen oder eines Tieres angebracht wurde, von aussen nicht sichtbar ist, ermöglicht dies so eine diskrete topische Verabreichung eines pharmazeutischen Wirkstoffes.

### Kurzbeschreibung der Zeichnungen

Die Figuren 1 bis 3 zeigen eine erste Ausführungsform des erfindungsgemässen Bandes und die Figuren 4a bis 5c eine zweite Ausführungsform des erfindungsgemässen Bandes.
Figur 1 zeigt einen Teil des erfindungsgemässen Bandes mit einer geöffneten zweiteiligen Kammer.
Figur 2 zeigt das erfindungsgemässe Band der Figur 1, wobei in die zweiteilige Kammer ein wirkstoffhaltiger Behälter eingelegt ist.

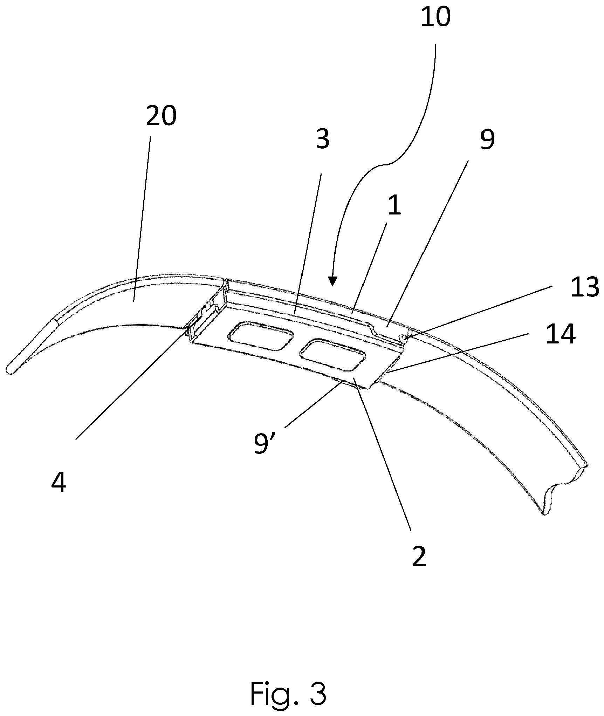

In dem in Figur 3 gezeigten Teil des Bandes ist die zweiteilige Kammer, in der der wirkstoffhaltige Behälter aufgenommen ist, geschlossen.

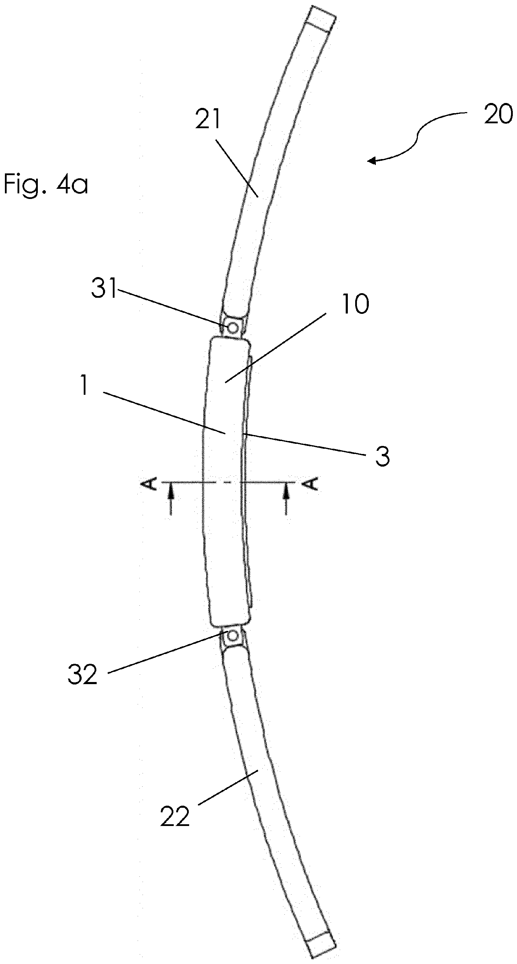

Die Figuren 4a bis 4c zeigen verschiedene Darstellungen eines erfindungsgemässen Bandes mit mehreren Bandabschnitten und einer einteiligen Kammer, in deren Kammerboden ein wirkstoffhaltiger Behälter eingeklemmt ist.

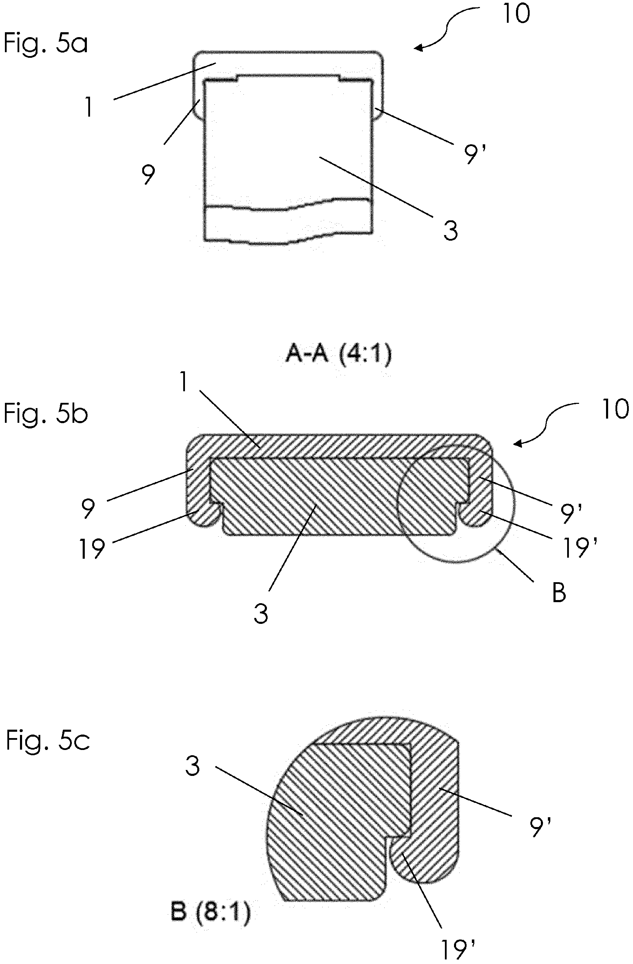

Die Figuren 5a bis 5c zeigen Details des in den Figuren 4a bis 4c gezeigten erfindungsgemässen Bandes.

### Bevorzugte Ausführungsformen der Erfindung

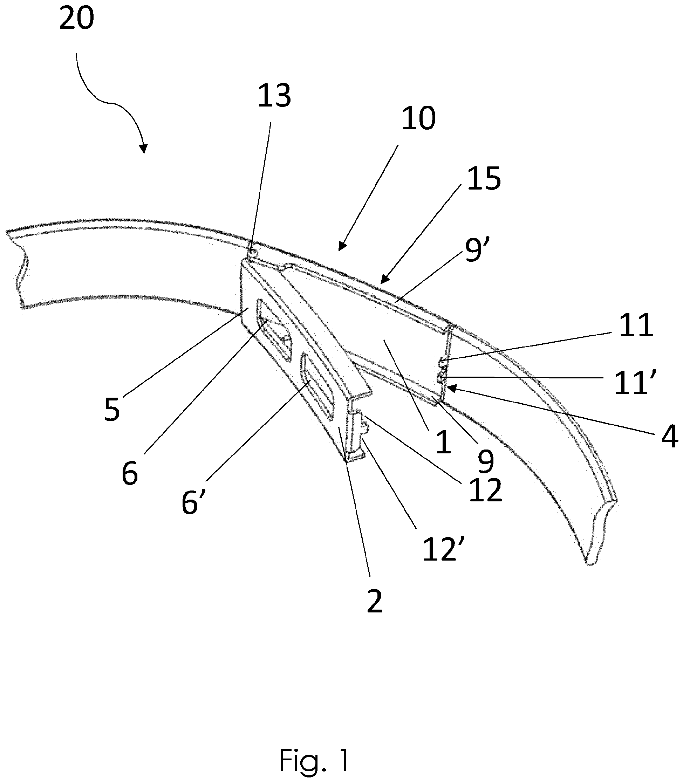

Wie in der Figur 1 gezeigt ist, weist das erfindungsgemässe Band 20 eine zweiteilige Kammer 10 zur Aufnahme eines wirkstoffhaltigen Behälters 3 (nicht dargestellt) auf. Die Kammer 10 weist einen zweiten Teil 2, auch als Deckel oder Kammerdeckel bezeichnet, und einen ersten Teil 1 auf, auch als Boden oder Kammerboden bezeichnet, zwischen denen der wirkstoffhaltige Behälter 3 eingebracht werden kann. Der Kammerdeckel 2 weist an seiner Fläche 5 Aussparungen 6, 6' für den formschlüssig dazu ausgebildeten wirkstoffhaltigen Behälter 3 auf. Der Kammerboden 1 begrenzt mit Seitenwänden 9, 9' zumindest teilweise die Aufnahme des wirkstoffhaltigen Behälters 3. Die zwei Teile 1,2 der Kammer sind an einer Seite über ein Scharnier 13 gelenkig miteinander und an der gegenüberliegenden Seite 4 mittels einer Rastverbindung lösbar miteinander verbunden. Erste Rastmittel 11, 1 1 ` des Kammerbodens 1 und zweite Rastmittel 12, 12' des Kammerdeckels 2 bilden die Rastverbindung, wenn die Kammer geschlossen ist, wie es in Figur 3 gezeigt ist.

Figur 1 zeigt auch die dem Kammerdeckel 2 abgewandte Fläche 15, die sogenannte Grundfläche, des Kammerbodens 1, über die die Kammer 10 bzw. der Kammerboden 1 am Band 20 befestigt sein kann. Hierbei können sowohl am Band 20 wie auch an der Grundfläche 15 jeweils komplementäre erste und zweite Befestigungsmittel (nicht dargestellt) angebracht sein.

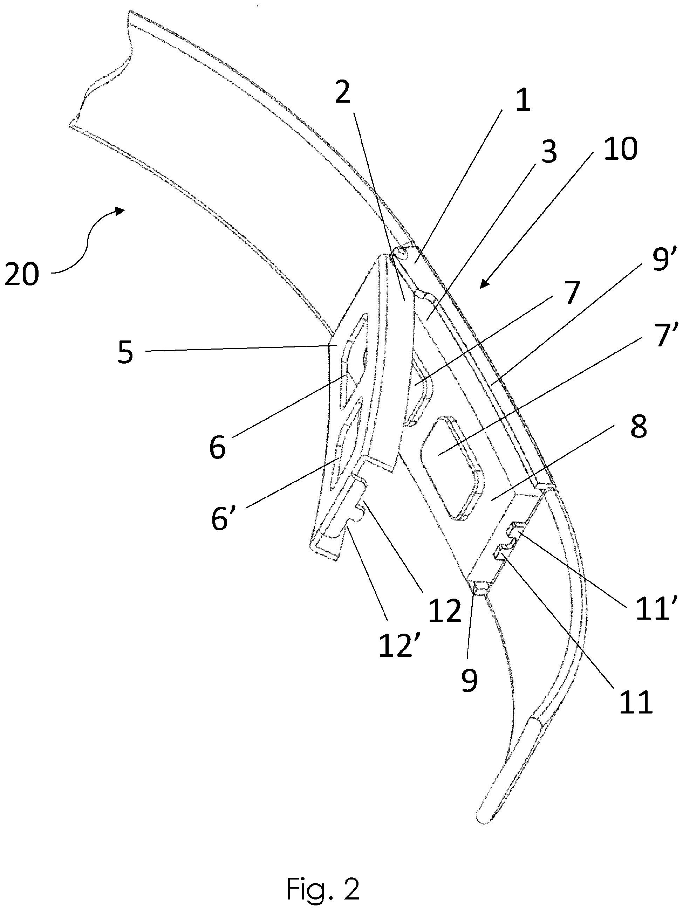

Wie in Figur 2 dargestellt, ist der wirkstoffhaltige Behälter 3 zwischen dem ersten Teil 1 der Kammer und dem zweiten Teil 2 der Kammer eingelegt. Die Fläche 5 des zweiten Teils 1 weist zwei Aussparungen 6, 6' auf, die formschlüssig zu zwei Erhebungen 7, 7' auf einer Fläche 8 des wirkstoffhaltigen Behälters 3 ausgebildet sind.

In Figur 3 ist die erste Seite 4 der Kammer 10 zu sehen, an der die beiden Teile 1, 2 der Kammer lösbar miteinander verrastet sind. Ferner sind die Seitenwände 9,9' des Kammerbodens gezeigt, die die passgenaue Aufnahme des wirkstoffhaltigen Behälters 3 ermöglichen. Das Scharnier 13 auf der zweiten Seite 14 der Kammer 10 ermöglicht ein Schwenken des Kammerdeckels 2 gegenüber dem Kammerboden 1.

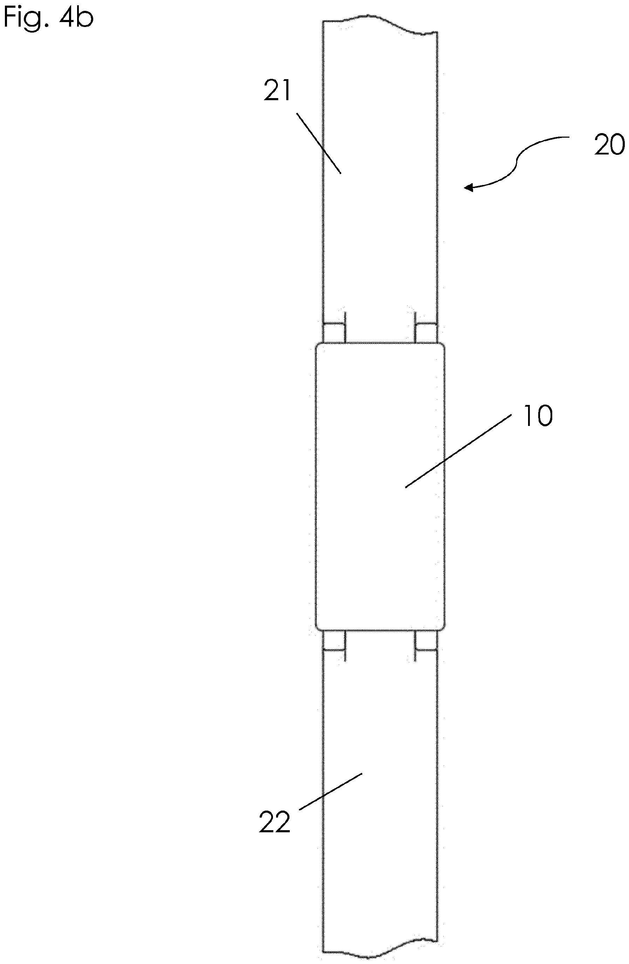

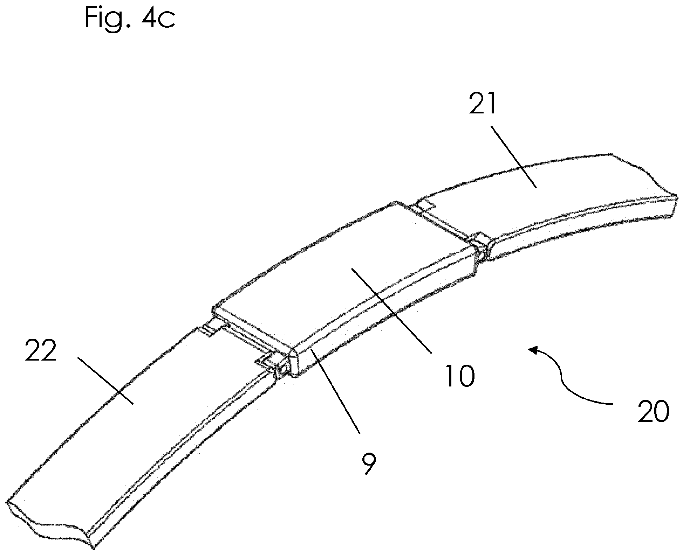

Das in den Figuren 4a bis 4c gezeigte Band 20 weist mehrere Bandabschnitte 21, 22 auf, die mit einer sich zwischen den Bandabschnitten befindlichen einteiligen Kammer 10 durch entsprechende Befestigungsmittel 31, 32 verbunden sind. In der gezeigten Ausführungsform sind die Befestigungsmittel 31, 32 Scharniere, so dass die einzelnen Bandabschnitte 21, 22 gegenüber der Kammer 10 beweglich sind. In den Kammerboden 1 der Kammer 10 ist ein wirkstoffhaltiger Behälter 3 eingeklemmt. Die Fig. 4a zeigt das Band in Seitenansicht, die Fig. 4b von oben und die Fig. 4c in Aufsicht.

Die Figuren 5a bis 5c zeigen Details des in den Figuren 4a bis 4c gezeigten erfindungsgemässen Bandes. Die Figuren 5a und 5b zeigen die Kammer 10 mit eingeklemmtem wirkstoffhaltigen Behälter 3 entlang der in Figur 4a eingezeichneten Achse A-A. Die Darstellung in Fig. 5b ist im Massstab 4:1. Die Figur 5c zeigt den in Fig. 5b eingezeichneten Ausschnitt B im Massstab 8:1.

Der wirkstoffhaltige Behälter 3 ist hier in den ersten Teil 1 der Kammer 10, den sogenannten Kammerboden 1, eingeklemmt, was durch die Seitenränder 9, 9' des Kammerbodens ermöglicht wird. Um ein leichtes Herausrutschen des wirkstoffhaltigen Behälters 3 zu verhindern, weisen die Seitenränder 9, 9' eine entsprechende Biegung 19, 19' auf. Bevorzugt ist der wirkstoffhaltige Behälter 3 ein Cannabidiol-haltiges Pad.

### Bezugszeichenliste

- 10: Kammer
- 20: Band
- 21,22,23: Bandabschnitt
- 31, 32, 33: Befestigungsmittel, beispielsweise Scharnier
- 1: erster Teil der Kammer, Boden, Kammerboden
- 2: zweiter Teil der Kammer, Deckel, Kammerdeckel
- 3: wirkstoffhaltiger Behälter
- 4: erste Seite der Kammer
- 5: Fläche des zweiten Teils der Kammer
- 6, 6`: Aussparung der Fläche 5
- 7, 7': Erhebung der Fläche 8
- 8: Fläche des wirkstoffhaltigen Behälters
- 9, 9': Seitenwand des ersten Teils der Kammer
- 11, 11': erste Rastmittel
- 12, 12': zweite Rastmittel
- 13: Scharnier
- 14: zweite Seite der Kammer
- 15: Grundfläche des ersten Teils der Kammer
- 19, 19': Biegung der Seitenwand 9, 9'

## Patentansprüche

1. Ein Band (20) umfassend mindestens eine Kammer (10), wobei die mindestens eine Kammer (10) einen ersten Teil (1) aufweist, wobei in die mindestens eine Kammer (10) ein wirkstoffhaltiger Behälter (3) einbringbar und aus der mindestens einen Kammer (10) ausbringbar ist, und wobei der wirkstoffhaltige Behälter (3) in Kontakt bringbar ist mit einem an die mindestens eine Kammer (10) anlegbaren Körperteil eines Menschen oder eines Tiers.

2. Das Band (20) nach Anspruch 1, wobei der wirkstoffhaltige Behälter (3) ein Cannabidiol-haltiges Pad ist.

3. Das Band (20) nach Anspruch 1 und/oder 2, wobei die Kammer zweiteilig ausgebildet ist, und zusätzlich einen zweiten Teil aufweist, wobei die zweiteilige Kammer öffen- und verschliessbar ist.

4. Das Band (20) nach einem der Ansprüche 1 bis 3, wobei der wirkstoffhaltige Behälter (3) entweder in den ersten Teil (1) der mindestens einen einteiligen Kammer oder zwischen den ersten Teil (1) und den zweiten Teil (2) der mindestens einen zweiteiligen Kammer einklemmbar ist.

5. Das Band (20) nach einem der Ansprüche 3 bis 4, wobei der erste Teil (1) der mindestens einen zweiteiligen Kammer und der zweite Teil (2) der mindestens einen zweiteiligen Kammer an mindestens einer Seite (4) lösbar miteinander verbunden sind.

6. Das Band (20) nach Anspruch 5, wobei der erste Teil (1) der mindestens einen zweiteiligen Kammer an einer dem zweiten Teil (2) der mindestens einen zweiteiligen Kammer zugewandten Seite erste Rastmittel (11, 1 1') aufweist, und wobei der zweite Teil (2) der mindestens einen zweiteiligen Kammer an einer dem ersten Teil (1) der mindestens einen zweiteiligen Kammer zugewandten Seite komplementäre zweite Rastmittel (12, 12') aufweist, und wobei die ersten Rastmittel (11, 11') und die zweiten Rastmittel (12, 12') eine Rastverbindung bilden, wobei die Rastverbindung zwischen den ersten Rastmitteln (11, 11') und den zweiten Rastmitteln (12, 12') bevorzugt formschlüssig ist.

7. Das Band (20) nach einem der Ansprüche 3 bis 6, wobei der zweite Teil (1) der mindestens einen zweiteiligen Kammer so ausgestaltet ist, dass der wirkstoffhaltige Behälter (3) darin einlegbar ist.

8. Das Band (20) nach einem der Ansprüche 3 bis 7, wobei der zweite Teil (2) der mindestens einen zweiteiligen Kammer eine Fläche (5) mit mindestens einer Aussparung (6) aufweist, und wobei die Aussparung (6) des zweiten Teils (2) der mindestens einen zweiteiligen Kammer so konfiguriert ist, dass mindestens eine Erhebung (7) auf der dem zweiten Teil (2) der mindestens einen zweiteiligen Kammer zugewandten Fläche (8) des wirkstoffhaltigen Behälters (3) formschlüssig zu der Aussparung (6) des zweiten Teils (1) der mindestens einen zweiteiligen Kammer ist.

9. Das Band (20) nach einem der Ansprüche 3 bis 8, wobei der zweite Teil (2) der mindestens einen zweiteiligen Kammer gegenüber dem ersten Teil (1) der mindestens einen zweiteiligen Kammer um einen Winkel von 0 bis mindestens 90° schwenkbar ist.

10. Das Band (20) nach einem der vorhergehenden Ansprüche, wobei das Band (20) öffen- und verschliessbar ist, und wobei bevorzugt eine der mindestens einen Kammer (10) als zweiteilige Kammer ausgebildet und als Verschluss des Bandes ausgestaltet ist.

11. Das Band (20) nach einem der vorhergehenden Ansprüche, wobei das Band (20) ein Armband für einen Menschen ist oder das Band (20) zur Befestigung an einem Körperteil eines nichthumanen Säugetiers ausgestaltet ist.

12. Das Band (20) nach Anspruch 11, wobei das Armband für einen Menschen als Schmuckstück und/oder Uhrenarmband ausgestaltet ist; oder
wobei das Band (20) ein Halsband, ein Beinband oder ein Halfter für ein nichthumanes Säugetier ist, wobei das nichthumane Säugetier Haustiere und Nutztiere umfasst, wobei das nichthumane Säugetier insbesondere Hunde, Katzen, Rinder, Schafe, Ziegen, Schweine, Kamele und Pferde umfasst.

13. Das Band (20) nach einem der vorhergehenden Ansprüche, wobei in die mindestens eine Kammer (10) ein wirkstoffhaltiger Behälter (3) eingebracht ist.

14. Das Band (20) nach Anspruch 13, wobei der Behälter gefüllt ist mit einem pharmazeutischen Wirkstoff und/oder dessen Formulierung und/oder einer biologisch aktiven Substanz und/oder deren Formulierung, wobei der pharmazeutische Wirkstoff insbesondere ausgewählt ist aus Pharmazeutika, welche in der Schmerztherapie und/oder in der Psychotherapie eingesetzt werden, wobei der pharmazeutische Wirkstoff bevorzugt ausgewählt ist aus Cannabinoiden wie Cannabidiol und Tetrahydrocannabinol sowie deren Mischungen und Derivaten wie Ethern und Estern, wobei der pharmazeutische Wirkstoff besonders bevorzugt Cannabidiol ist.

15. Verwendung eines Bandes (20) nach einem der vorhergehenden Ansprüche zur topischen Verabreichung eines Wirkstoffes und/oder dessen Formulierung und/oder einer biologisch aktiven Substanz und/oder deren Formulierung an einen Menschen oder ein Tier, wobei die Verwendung insbesondere nicht-medizinisch ist.
